(19) Europäisches Patentamt
European Patent Office
Office européen des brevets

(11) Veröffentlichungsnummer: **0 186 807 B1**

# (12) EUROPÄISCHE PATENTSCHRIFT

(45) Veröffentlichungstag der Patentschrift: **16.08.89**

(51) Int. Cl.⁴: **C 07 H 15/252**, C 07 C 50/38, A 61 K 31/70, A 61 K 31/12, C 12 P 15/00 // (C12P19/56, C12R1:465),(C12P15/00, C12R1:465)

(21) Anmeldenummer: **85115490.6**

(22) Anmeldetag: **05.12.85**

(54) **Anthracyclin-Derivate, ein mikrobiologisches Verfahren zu ihrer Herstellung und ihre Verwendung als Arzneimittel.**

(30) Priorität: **18.12.84 DE 3446052**

(43) Veröffentlichungstag der Anmeldung: **09.07.86 Patentblatt 86/28**

(45) Bekanntmachung des Hinweises auf die Patenterteilung: **16.08.89 Patentblatt 89/33**

(84) Benannte Vertragsstaaten: **AT BE CH DE FR GB IT LI LU NL SE**

(73) Patentinhaber: **HOECHST AKTIENGESELLSCHAFT, Postfach 80 03 20, D-6230 Frankfurt am Main 80 (DE)**

(72) Erfinder: **Franco, Christopher Milton Mathew, Dr., 74A "The Westminister" Duncan Causeway, Sion Bombay 400 022 (IN)**
Erfinder: **Mukhopadhyay, Triptikumar, Dr., 15, A-Wing, "Hansa Sagar" Govardhan Nagar, L.B.S. Marg Mulund (West) Bombay 400 080 (IN)**
Erfinder: **Desikan, Kalyanapuram Rajagopalan, 16, "Abhilasha" Off Dr. R.P. Road, Mulund (West) Bombay 400 080 (IN)**
Erfinder: **Ganguli, Bimal Naresh, Dr., "Saurayuth" 173 Central Avenue, Chembur Bombay 400 071 (IN)**
Erfinder: **Fehlhaber, Hans-Wolfram, Dr., Thomas-Mann-Strasse 5a, D-6270 Idstein/Taunus (DE)**
Erfinder: **Kraemer, Hans Peter, Dr., Birkenweg 16, D-3550 Marburg (DE)**

(56) Entgegenhaltungen:
**EP-A- 0 078 447**
**GB-A- 2 048 245**

**CHEMISCHE BERICHTEN, Band 113, Nr. 9, 1980, Seiten 2976-2993, Verlag Chemie GmbH, Weinheim, DE. K. KROHN et al.: "Synthetische Anthracyclinone, XIV: Synthese neuer Derivate des Daunomycinons und des beta-Rhodomycinons"**
**CHEMISCHE BERICHTEN, Band 113, Nr. 9, 1980, Seiten 2994-3009, Verlag Chemie GmbH, Weinheim, DE. K.**

(56) Entgegenhaltungen: (Fortsetzung)
**KROHN et al.: "Synthetische Anthracyclinone, XV. Regio- und stereoselektive Synthese der alpha-, beta- und gamma-Rhodomycicone über intramolekulare Marschakk-Cyclisierung"**
**JOURNAL OF ANTIBIOTICS, Band 34, Nr. 12, Dezember 1981, Seiten 1596-1607, Tokyo, JP. Y. MATSUZAWA et al.: "Structure-activity relationships of anthracyclines relative to cytotoxicity and effects on macromolecular synthesis in L1210 leukemia cells"**

EP 0 186 807 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft neue, nachfolgend als Kesarirhodine oder Säureadditionssalze von Kesarirhodin bezeichnete Anthracyclin-Verbindungen sowie ein Verfahren zu ihrer Herstellung, das dadurch gekennzeichnet ist, dass man den Mikroorganismus (HPL Y-11 472) – DSM 2658 in Gegenwart von chemischen Inhibitoren fermentiert.

Die neuen Verbindungen besitzen eine antibakterielle Wirkung gegen gram-positive Bakterien und weisen ausserdem eine Wirkung gegen eine Reihe von Tumorarten auf. Die neuen Verbindungen besitzen die in der nachfolgenden Formel I dargestellte allgemeine Struktur:

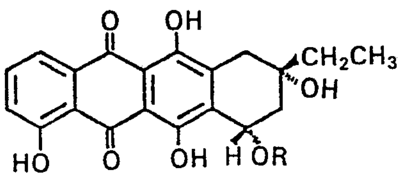

I

in der R für eine Zuckerkombination folgender Art steht:

(A) Roa-dF-Rod (B) Roa-Rod-Rod,

wobei Roa Rhodosamin, dF Deoxyfucose und Rod Rhodinose darstellt.

Die Struktur dieser Zucker ist in den nachfolgenden Formelbildern dargestellt.

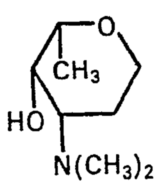

Rhodosamin (Roa)

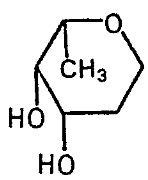

Deoxyfucose (df)

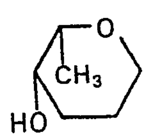

Rhodinose (Rod)

Die beiden Typen der Kesarirhodine entsprechend den Zuckerkombinationen (A) und (B) werden als Kesarirhodin A bzw. Kesarirhodin B bezeichnet. Dementsprechend bedeutet «Kesarirhodin» in der vorliegenden Erfindung entweder Kesarirhodin A oder B oder eine Mischung beider Kesarirhodine. Kesarirhodin besitzt in seinem Saccharidanteil eine Dimethylamino-Gruppe und kann damit Säureadditionssalze bilden. Zu den Säuren, die durch Zusatzreaktion mit Kesarirhodin Salze bilden, zählen z.B. Salzsäure, Schwefelsäure und Weinsäure.

Der Mikroorganismus zur Herstellung von Kesarirhodinen Streptomyces purpurascens (HPL Y-11 472) – DSM 2658 gehört zur Klasse Actinomycetales, Familie Streptomycetaceae und Genus Streptomyces. Der Mikroorganismus wird nachstehend als «S. purpurascens» – DSM 2658 oder «produzierender Mikroorganismus» bezeichnet.

Anthracyclin-Antibiotika werden nachgewiesenermassen von verschiedenen Streptomyces-Spezies produziert und sind in der Literatur beschrieben worden. Diese Antibiotika spielen in der Medizin für die Beherrschung von Tumoren in der Krebstherapie und als antibakterielle Medikamente eine wichtige Rolle. Bisher sind verschiedene Anthrazyklin-Verbindungen vorgestellt worden. Von diesen sind bisher in der Klinik bei Karzinomen Daunomycin und Adriamycin angewandt worden.

In GB-A-2 048 245 ist die Verbindung 13-Deoxycarminomycin beschrieben, dessen Aglykon dem der erfindungsgemässen Verbindungen entspricht, der Aminozucker in 7-Stellung jedoch Daunosamin darstellt.

Chem. Ber. 113, 2994–3009 (1980) betrifft die Synthese von Rhodomycinonen, die jedoch als Aglykone keine Zuckermoleküle tragen.

In EP-A-78 447 werden β-Rhodomycinon-Glykoside mit den Zuckerkombinationen Roa-dF-A, Roa-dF-Rod und Roa-dF-CinA in 7-Stellung sowie einer OH-Gruppe in 10-Stellung beschrieben.

Rhodomycine, Iso-Rhodomycine und vom Rhodomycin abgeleitete Anthrazyklin-Antibiotika sind z.B. in J. Med. Chem. 20, 957–960 (1977) beschrieben. Aclacinomycin ist ausführlich im U.S. Patent No. 3 988 315 und von Oki et al. in J. Antibiotics 28, 830 (1975) und 32, 791–812 (1979) beschrieben worden.

Die Cinerubine A und B werden im U.K. Patent No. 846 130, U.S. Patent No. 3 864 489 und von Keller-Schlierlein et al. in «Antimicrobial Agents and Chemotherapy», Seite 68 (1970), Chemical Abstracts 54, 14 661 (1960) und J. Antibiotics 28, 830 (1975) beschrieben.

Weitere zusammenfassende Beschreibungen der Anthracyclin-Antibiotika können im «Index of Antibiotics from Actinomycetes», Hamao Umezawa, Editor-in-Chief, University Park Press, State College, Pennsylvania, U.S.A. (1976) wie folgt nachgelesen werden:

| Antibiotika | Seiten |
|---|---|
| Aclacinomycine A-B | 101–102 |
| Adriamycin | 124 |
| Carminomycin I | 225 |
| Galirubine S-D | 405–408 |
| Rhodomycine X-Y | 879–880 |
| β-Rhodomycine | 881–885 |
| γ-Rhodomycine | 886–892 |
| Steffimycin | 945 |

Die vorliegende Erfindung betrifft auch ein Verfahren zur Herstellung von Verbindungen, die nachstehend als Kesarirhodine bezeichnet werden, zur Klasse der Anthracycline zählen und die allgemeine Formel I besitzen, mittels Kultivierung

von S.purpurascens – DSM 2658 durch Fermentierung bei einem pH-Wert zwischen 6,5 und 8,5 und einer Temperatur zwischen 24 °C bis 40 °C unter aeroben Bedingungen in einem Nährmedium, das Kohlenstoff und Stickstoff liefert, als Nährstoffe anorganische Salze und Spurenelemente enthält, in Abwesenheit oder Gegenwart von Antischaummitteln wie Silikonöl oder Desmorphen, jedoch in Anwesenheit von chemischen Inhibitoren, und die Isolierung der Verbindungen aus Kulturflüssigkeit und Mycelium in der nachstehend beschriebenen Weise.

Kohlenstoffquellen können Glukose, Saccharose, Stärke, Glyzerin, Dextrin, Fruktose, Melassen, Hafermehl, Maltose, Laktose und Galaktose sein. Bevorzugte Kohlenstoffquellen sind Glukose, Stärke und Saccharose. Stickstoffquellen können Sojabohnenmehl, Hefemehl, Hefeextrakt, Rinderextrakt, Malzextrakt, Agar, Pepton, Kasein, Baumwollsamenöl, Kichererbsensamenpulver und anorganische Verbindungen wie Ammoniumsalze oder Nitrate (z.B. Ammoniumsulfat, Natriumnitrat, Ammoniumchlorid und Kaliumnitrat) sein. Bevorzugte Stickstoffquellen sind Malzextrakt, Hefeextrakt, Sojabohnenmehl und Kaliumnitrat. Falls erwünscht, können Nährstoffe in Form anorganischer Salze wie Natriumchlorid, Magnesiumsulfat, Kaliumchlorid, Kaliumhydrogen- und -dihydrogenphosphat, Calciumchlorid, Calciumcarbonat, Ammoniumhydrogenphosphat, Natriumhydrogen- und -dihydrogenphosphat, Magnesiumphosphat und Calciumphosphat zugesetzt werden. Als Spurenelemente können Eisen-, Mangan-, Kupfer-, Zink- und andere Schwermetallsalze verwendet werden. Als chemische Inhibitoren können Kaliumcyanid, Kaliumhexacyanoferrat (III), Methylenblau, Triphenyltetrazoliumchlorid, Sulfanilsäure, Sulfanilamid, Ascorbinsäure und ihre Salze, Natriumazid, Eisen(III)chlorid, Äthylendiaminotetraessigsäure, Nitrofurazon, Salinomycin, Dithionit, Rotenon, 2,4-Dinitrophenol, Erythromycin und Dimethylsulfoxid dienen. Bevorzugte Inhibitoren sind Ascorbinsäure und deren Salze, Natriumazid und Eisen(III)chlorid.

Die Kultivierung von S. purpurascens – DSM 2658 kann bei Temperaturen zwischen 24 °C bis 40 °C und einem pH-Wert zwischen 6,5 und 8,5 erfolgen. S. purpurascens DSM wird vorzugsweise unter aeroben Bedingungen bei 27 °C und pH-Wert 7,0 kultiviert. Der chemische Inhibitor wie Eisen(III)chlorid, Natriumazid oder Ascorbinsäure kann nach 20–36 Stunden Fermentierung zugesetzt werden, um eine Endkonzentration zwischen 10–80 Milligramm pro Liter der Fermentierungsbrühe zu erreichen. Vorzugsweise wird nach 27 Stunden Fermentierung Natriumazid zugesetzt, um eine Endkonzentration von 20 Milligramm pro Liter zu erreichen. Die Fermentierung wird nach 48 bis 56 Stunden abgebrochen, wenn optimale Mengen der gewünschten Verbindungen gewonnen worden sind. Als Fermentierung wird die Submersfermentierung bevorzugt. Zu Beginn der Fermentierung kann dem Fermenter bis zu einer Endkonzentration von 0,025% das Antischaummittel Desmophen(R) zugesetzt werden.

In den so entstandenen Kulturbrühen ist das angesammelte Kesarirhodin sowohl in Mycelium wie im Kulturfiltrat enthalten.

Der Verlauf der Fermentation und die Bildung der Anthracyclin-Verbindungen lassen sich durch Extrahieren der Gesamtbrühe (Mycelium und Kulturfiltrat) mit einem organischen Lösungsmittel und Messung der Absorptionsintensität bei 492 nm und durch Chromatographie des Extraktes auf Kieselsäureplatten sowie durch die antibakterielle Wirkung gegen Staphylococcus aureus 209 P nachweisen. Als organische Lösungsmittel können Chloroform, Äthylacetat, Butylacetat, Butanol, Toluol und Benzol verwendet werden. Äthylacetat wird bevorzugt.

Die Kesarirhodine können durch jede zu ihrer Gewinnung aus diesen Kulturbrühen geeignete Methode gewonnen werden. Eine dieser Methoden, die in dem beigefügten Schema I dargestellt ist, basiert auf der Extraktion. So kann Kesarirhodin im Kulturfiltrat beispielsweise durch die Extraktion mit einem mit Wasser unvermischbaren Lösungsmittel wie Äthylacetat gewonnen werden, nachdem der pH-Wert des Filtrates auf 7,5–8,0 eingestellt worden ist.

Kesarirhodin im Mycelium kann durch die Behandlung des Myceliums, das durch Filtration oder Zentrifugieren gewonnen worden ist, mit Äthylacetat, Chloroform, Methanol, Äthanol, Aceton, Butanol, Methyläthylketon, eine Salzsäurelösung oder eine Essigsäurelösung isoliert werden. Als Lösungsmittel wird Aceton bevorzugt. Nach der Extraktion wird das Aceton entfernt und die wässrige Schicht auf einen pH-Wert von 7,5–8,0 eingestellt und mit Äthylacetat extrahiert. Man kann die Kulturbrühe auch den oben beschriebenen Extraktionsschritten unterziehen, ohne das Mycelium abzutrennen.

Die Äthylacetat-Extrakte des Kulturfiltrats und des Mycelium werden kombiniert oder getrennt aufbereitet, konzentriert und dann nach Schema II gereinigt.

Schema I:
Isolierung des antibiotischen Komplexes aus S. purpurascens DSM 2658

Fermentierungsbrühe

| Mycelium | | Kulturfiltrat | |
|---|---|---|---|
| I. | Verrühren mit Aceton: Acetatpuffer (9:1) pH-Wert 3,5, 2 Std. lang | I. | pH-Wert auf 7,5 einstellen |
| II. | Konzentrieren und Extrahieren mit Äthylacetat bei pH-Wert 7,5 | II. | Extrahieren mit Äthylacetat |

Wässrige Schicht | Äthylacetat-Schicht | Wässrige Schicht wegwerfen

Äthylacetat-Schicht
↓
Eindampfen bis zur Trockene
↓
Rohextrakt – FRAKTION K

Schema II:
Isolieren und Reinigen von Kesarirhodin A und B

Rohextrakt: Fraktion K
↓
Dünnschicht-Chromatographie-Kieselgel-Säule (4,5×40 cm)
↓
Eluieren mit Lösungsmittel A
↓
Halbreine Fraktion A | Halbreine Fraktion B | Fraktion 'X'

Halbreine Fraktion A:
I. Mit Petroläther waschen
II. Präparative Dünnschicht-Chromatographie/Kieselgel/Lösungsmittel A
↓
Kesarirhodin A

Halbreine Fraktion B:
I. Mit Petroläther waschen
II. Präparative Dünnschicht-Chromatographie/Kieselgel/Lösungsmittel A
↓
Kesarirhodin B

Lösungsmittel A= Chloroform:Methanol:Essigsäure:Aqua dest.:Triäthanolamin (80:10:10:2:0,1)

Eine andere Methode zur Gewinnung von Kesarirhodin aus der Kulturbrühe basiert auf der Adsorption. Das Kesarirhodin enthaltende flüssige Material wie z.B. das Kulturfiltrat oder der durch das vorstehend beschriebene Extraktionsverfahren gewonnene Extrakt werden der Säulenchromatographie, Flüssigkeitschromatographie oder einer anderen Methode unterzogen, bei der ein geeignetes Adsorbens wie Aktivkohle, Diaion HP-20$^{(R)}$, XAD$^{(R)}$, Aluminiumoxid, Kieselgel oder Sephadex LH-20$^{(R)}$ verwendet werden. Die Kesarirhodine können mit Methanol oder Aceton aus den Adsorbentien eluiert werden. Die gewonnenen Eluate werden bis zur Trockene konzentriert, wobei rohes Kesarirhodin als orangerotes Pulver anfällt. Die rohen Kesarirhodine können gereinigt werden, indem man die vorstehend beschriebene Extraktions- und Adsorptionstechnik beliebig oft wiederholt. Verwendet werden können je nach Wunsch die Säulenchromatographie mit den bereits erwähnten verschiedenen Absorbentien, die Gegenstromverteilung oder die präparative Dünnschichtchromatographie und Kristallisation. Die weitere Reinigung lässt sich mittels Hochleistungs-Flüssigkeitschromatographie erreichen.

Wie in Schema II dargestellt ist, liefert die Fraktion K drei Komponenten, von denen die halbreine Fraktion A weiter gereinigt wird, um eine reine Verbindung zu liefern, die nachstehend als Kesarirhodin A bezeichnet wird. Die Fraktion B wird weiter gereinigt, um eine reine Verbindung zu liefern, die nachstehend als Kesarirhodin B bezeichnet wird. Die Fraktion 'X' stellt eine Mischung aus zusätzlichen Anthracyclin-Verbindungen dar, die noch weiter untersucht werden. Die allgemeine Struktur der Kesarirhodine ist mit der in der Formel I identisch.

Die aus der Fermentierungsbrühe von S. purpurascens DSM 2658 isolierten und wie in Schema I und II gereinigten Kesarihodine A und B besitzen die nachstehend dargestellten Strukturformeln Ia und Ib:

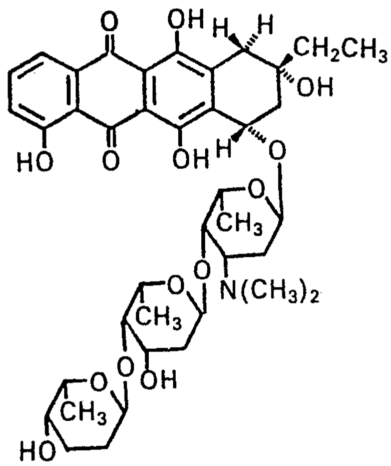

Kesarirhodin A

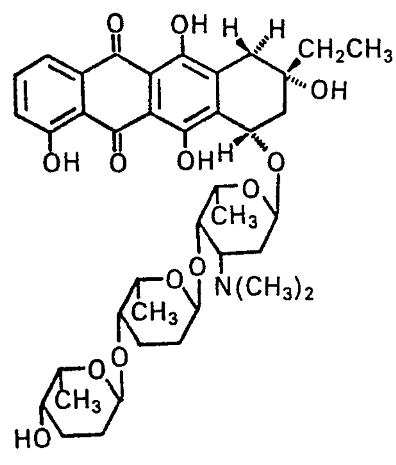

Kesarirhodin B

Die Kesarirhodin-Antibiotika gemäss dieser Erfindung besitzen antibakterielle Wirkung gegen gram-positive Bakterien, fungizide Wirkung und Antitumor-Wirkung.

Die Erfindung wird durch die nachfolgenden Beispiele illustriert.

Beispiel 1:
Kultivierung von S. purpurasens – DSM 2658 für die fermentative Produktion des antibiotisch wirksamen Komplexes

Streptomyces purpurascens – DSM 2658 wird auf Hefe-Malz-Agar mit folgender Zusammensetzung gehalten:

| | |
|---|---|
| Malzextrakt | 10,0 g |
| Hefeextrakt | 4,0 g |
| Glucose | 4,0 g |
| Maisquellwasser | 15,0 g |
| Agua dest. | 1 Liter |
| pH | 7,0 |

Das Medium wird in Reagenzgläser verteilt und 20 Minuten lang bei 121 °C sterilisiert. Die Reagenzgläser werden in Schräglage zur Herstellung von Schrägagarröhrchen abgekühlt. Die Schrägagarröhrchen werden mit der Kultur geimpft und 10–15 Tage lang bei 28 °C inkubiert, bis gutes Wachstum und Sporenbildung zu beobachten sind. Eine Sporensuspension in Aqua dest. von einem Schrägagarröhrchen wird dazu verwendet, fünf Erlenmeyer-Kolben zu 500 ml, die je 100 ml des Impfkulturmediums enthalten, oder eine Aspiratorflasche zu 5 Liter, die 1 Liter des gleichen Impfkulturmediums enthält, zu inokulieren.

Zusammensetzung des Impfkulturmediums

| | |
|---|---|
| Glucose | 15,0 g |
| Sojabohnenmehl | 15,0 g |
| Maisquellwasser | 5,0 g |
| $CaCO_3$ | 2,0 g |
| NaCl | 5,0 g |
| Aqua dest. | 1 Liter |
| pH | 7,0 |

Das vorgenannte Medium wird in Mengen von 100 ml auf Erlenmeyer-Kolben zu 500 ml oder in Mengen von 1 Liter auf Saugflaschen zu 5 Liter verteilt und 20 Minuten lang bei 121 °C sterilisiert. Die Kolben/Flaschen werden abgekühlt, mit der Sporensuspension beimpft und 72 Stunden lang bei 28 °C bei 240 Umdrehungen auf einem Drehschüttler mit einer Amplitude von 3,75 cm geschüttelt. Mit der gut angewachsenen Impfkultur wird ein 15 Liter-Glasfermenter beimpft, der 10 l des Produktionsmediums in einer Konzentration von 5 Vol.-% enthält.

Zusammensetzung des Produktionsmediums

| | |
|---|---|
| Glucose | 20,0 g |
| Malzextrakt | 10,0 g |
| Hefeextrakt | 4,0 g |
| Aqua dest. | 1 Liter |
| pH | 7,0 |

0,025% Desmorphen(R) werden als Antischaummittel dem Inhalt des Fermenters zugesetzt.

Vor dem vorstehend beschriebenen Medium werden 10 l in einen 15 l-Fermenter gegeben. Das Medium wird mittels indirektem und direktem Dampf 28 Minuten lang bei 121 °C sterilisiert. Der Fermenter wird abgekühlt und mit der Zweitstufen-Impfkultur (5 Vol.-%) inokuliert. Die Fermentierung erfolgt bei 27 °C (± 0,51 °C) unter Rühren bei 170–180 Umdrehungen 48–56 Stunden lang. Die Belüftung erfolgt mit einer Rate von 0,6–0,8 vvm. Im Verlauf der Fermentierung wird nach etwa 24–32 Stunden wie nachstehend beschrieben die Inhibitorsubstanz zugegeben (Beispiele 6–9).

Die Fermenter werden nach Ablauf von 48–56 Stunden abgeerntet, wenn die Konzentration des antibiotisch wirksamen Komplexes 30 50 Mikrogramm pro ml beträgt und der pH-Wert der Kulturflüssigkeit zwischen 5,5–6,0 liegt. Das Schleuder-Zellvolumen beträgt zu diesem Zeitpunkt 11–13 ml pro 100 ml.

Beispiel 2:
Kultivierung von S. purpurascens – DSM 2658 für die fermenative Produktion des antibiotisch wirksamen Komplexes

Die Kultur wird unter den gleichen Bedingungen wie in Beispiel 1 mit den folgenden Medien gezüchtet:

(1) Zusammensetzung des Impfkulturmediums:

| | |
|---|---|
| Kichererbsensamenpulver | 10,0 g |
| Dextrose | 10,0 g |
| NaCl | 5,0 g |
| $CaCO_3$ | 3,0 g |
| Aqua dest. | 1 Liter |
| pH | 7,0 |

72 Stunden bei 28 °C

Zusammensetzung des Produktionsmediums

| | |
|---|---|
| Stärke | 10,0 g |
| Glucose | 10,0 g |
| Malzextrakt | 7,5 g |
| Pepton | 7,5 g |
| NaCl | 3,0 g |
| $MgSO_4 \cdot 7H_2O$ | 1,0 g |
| $KH_2PO_4$ | 2,0 g |
| $CuSO_4 \cdot 5H_2O$ | 7,0 mg |
| $FeSO_4 \cdot 7H_2O$ | 1,0 mg |
| $MnSO_4 \cdot 4H_2O$ | 8,0 mg |
| $ZnSO_4 \cdot 7H_2O$ | 2,0 mg |
| Aqua dest. | 1 Liter |
| pH | 7,0 |

Im Verlauf der Fermentierung wird wie nachstehend beschrieben nach etwa 24–32 Stunden der chemische Inhibitor zugegeben (Beispiele 6–9). Die Fermenter werden nach Ablauf von 48–56 Stunden abgeerntet, wenn die Konzentration des antibiotisch wirksamen Komplexes 20–40 Mikrogramm pro ml beträgt und der pH-Wert der Kulturflüssigkeit zwischen 5,8–6,2 liegt. Zum Zeitpunkt der Ernte liegt das abgesetzte Zellvolumen bei 11–13 ml pro 100 ml.

Beispiel 3:
Kultivierung von S. purpurascens – DSM 2658 für die fermentative Produktion des Antibiotikakomplexes

Die Kultur wird unter den gleichen Bedingungen wie in Beispiel 1 mit folgenden Medien gezüchtet:

Zusammensetzung des Impfkulturmediums

| | |
|---|---|
| Malzextrakt | 10,0 g |
| Hefeextrakt | 4,0 g |

| | |
|---|---|
| Glucose | 4,0 g |
| Aqua dest. | 1 Liter |
| pH | 7,0 |
| 72 Stunden bei 28 °C | |

Zusammensetzung des Produktionsmediums

| | |
|---|---|
| Stärke | 10,0 g |
| Glucose | 10,0 g |
| Sojabohnenmehl | 15,0 g |
| $K_2HPO_4$ | 1,0 g |
| $MgSO_4 \cdot 7H_2O$ | 1,0 g |
| NaCl | 3,0 g |
| $CuSO_4 \cdot 5H_2O$ | 7,0 g |
| $FeSO_4 \cdot 7H_2O$ | 1,0 mg |
| $MnCl_2 \cdot 4H_2O$ | 8,0 mg |
| $ZnSO_4 \cdot 7H_2O$ | 2,0 mg |
| Aqua dest. | 1 Liter |
| pH | 7,0 |

Im Verlauf der Fermentierung wird nach etwa 24–32 Stunden wie nachstehend beschrieben der chemische Inhibitor zugesetzt (Beispiel 6–9). Die Fermentierung wird nach Ablauf von 48–52 Stunden abgebrochen.

Beispiel 4:
Kultivierung von S. purpurascens – DSM 2658 für die fermentative Produktion des antibiotisch wirksamen Komplexes

Die Kultur wird unter den gleichen Bedingungen wie in Beispiel 1 mit den folgenden Medien gezüchtet:

Zusammensetzung des Stammhaltung-Agars

| | |
|---|---|
| Hafermehl | 20,0 g |
| $FeSO_4 \cdot 7H_2O$ | 0,1 mg |
| $ZnSO_4 \cdot 7H_2O$ | 0,1 mg |
| $MnCl_2 \cdot 4H_2O$ | 0,1 mg |
| Aqua dest. | 1 Liter |
| pH | 7,2 |

Zusammensetzung des Impfkulturmediums

| | |
|---|---|
| Glucose | 10,0 g |
| Stärke | 10,0 g |
| Sojabohnenmehl | 15,0 g |
| NaCl | 3,0 g |
| $MgSO_4 \cdot 7H_2O$ | 1,0 g |
| $K_2HPO_4$ | 1,0 g |
| $CuSO_4 \cdot 5H_2O$ | 7,0 mg |
| $FeSO_4 \cdot 7H_2O$ | 1,0 mg |
| $MnCl_2 \cdot 4H_2O$ | 8,0 mg |
| $ZnSO_4 \cdot 7H_2O$ | 2,0 mg |
| Aqua dest. | 1 Liter |
| pH | 7,2 |
| 72 Stunden bei 28 °C | |

Zusammensetzung des Produktionsmediums

| | |
|---|---|
| Saccharose | 20,0 g |
| $CaCO_3$ | 2,5 g |
| $KNO_3$ | 1,0 g |
| $K_2HPO_4$ | 0,5 g |
| $MgSO_4 \cdot 7H_2O$ | 0,5 g |
| NaCl | 0,5 g |
| $FeSO_4 \cdot 7H_2O$ | 1,0 mg |
| Aqua dest. | 1 Liter |
| pH | 7,0 |

Im Verlauf der Fermentierung wird nach etwa 24–32 Stunden der chemische Inhibitor wie nachstehend beschrieben zugesetzt (Beispiele 6–9). Die Fermenter werden nach Ablauf von 48–52 Stunden abgeerntet, wenn die Konzentration des antibiotisch wirksamen Komplexes 25–40 Mikrogramm pro ml beträgt und der pH-Wert der Kulturflüssigkeit zwischen 6,3–6,4 schwankt. Zum Zeitpunkt der Ernte beträgt das abgesetzte Zellvolumen 5,0–6,0 ml pro 100 ml.

Beispiel 5:
Kultivierung von S. purpurascens – DSM 2658 für die fermentative Produktion des antibiotisch wirksamen Komplexes

Die Kultur wird auf die gleiche Weise wie in Beispiel 1 mit den folgenden Medien gezüchtet:

Zusammensetzung des Impfkulturmediums

| | |
|---|---|
| Glucose | 20,0 g |
| Sojabohnenmehl | 10,0 g |
| Aqua dest. | 1 Liter |
| pH | 7,2 |
| 72 Stunden bei 28 °C | |

Beispiel 6:
Zugabe des chemischen Inhibitors

Es wird eine konzentrierte Lösung von Ascorbinsäure oder deren Salzen (20 mg/ml) in Aqua dest. hergestellt und durch eine Sterilfiltration sterilisiert. Sie wird, wie in den Beispielen 1–5 beschrieben, dem Fermentationsmedium etwa 24–32 Stunden nach begonnener Fermentierung zugesetzt, wobei eine Endkonzentration von 20–100 Milligram Substanz pro Liter erreicht wird.

Beispiel 7:
Zugabe eines chemischen Inhibitors

Natriumazid wird in konzentrierter (20 mg/ml) steriler wässriger Lösung dem Fermentationsmedium, wie in den Beispielen 1–5 beschrieben, etwa 24–32 Stunden nach begonnener Fermentierung zugesetzt, wobei eine Endkonzentration von 10–80 mg Substanz pro Liter erreicht wird.

Beispiel 8:
Zugabe eines chemischen Inhibitors

Eisen(III)chlorid wird bis zu einer Konzentration von 20 mg/ml in Wasser gelöst, die Lösung durch Filtration sterilisiert und dem in den Beispielen 1–5 beschriebenen Fermentationsmedium etwa 24–32 Stunden nach begonnener Fermentierung zugesetzt, um eine Endkonzentration von 10–100 mg Substanz/Liter zu liefern.

Beispiel 9:
Zugabe eines chemischen Inhibitors

Der Zusatz eines Sulfonamids wie Sulfanilamid, Sulfacetamid oder Sulfonamid erfolgt durch Zugabe einer sterilen konzentrierten (20 mg/ml) wässrigen Lösung zu dem in den Beispielen 1-5

beschriebenen Fermentationsmedium etwa 24–32 Stunden nach Beginn der Fermentierung, wobei eine Endkonzentration von 40–200 mg Substanz pro Liter erreicht wird.

Beispiel 10:
Isolierung der Kesarirhodine

Es werden ca. 40 Liter der Fermentationsbrühe zentrifugiert um Mycelium und Kulturfiltrat voneinander zu trennen. Der Isolierungsvorgang von Kesarirhodin aus Mycelium und Kulturfiltrat ist in Schema I ausführlich dargestellt.

(a) Das Kulturfiltrat wird mit $NaHCO_3$ auf einen pH-Wert von 7,5 eingestellt und zweimal mit 20 Litern Äthylacetat extrahiert, die kombinierten Äthylacetat-Extrakte werden dann im Vakuum bis zur Trockene konzentriert. Man erhält ca. 1,5 g Extrakt, der als Fraktion K bezeichnet wird.

(b) Das abgetrennte Mycelium wird dreimal mit 20 Litern Aceton:Acetat-Puffer (9:1) bei pH 3,5 extrahiert, die kombinierten Extrakte unter Vakuum konzentriert, um das Aceton zu entfernen. Die zurückbleibende wässrige Schicht wird mit $NaHCO_3$ auf einen pH-Wert von 7,5 eingestellt und dreimal mit 30 Litern Äthylacetat extrahiert. Die kombinierten Extrakte werden unter Vakuum bis zur Trockene konzentriert. Man erhält ca. 6,0 g Extrakt, der als Fraktion K bezeichnet wird.

Der Kesarirhodin enthaltende Extrakt, d.h. die Fraktion K, aus Mycelium und Kulturfiltrat, kann zusammen oder getrennt aufbereitet werden. Der Isolierungsprozess von Kesarirhodin A und B ist in Schema II dargestellt.

(c) 7,5 g Extrakt (Fraktion K) werden auf eine 4,5×40 cm Dünnschicht-Chromatographie-Kieselgel-H-(BDH)-Säule gegeben und mit der folgenden Lösungsmittelmischung eluiert: Chloroform:Methanol:Essigsäure:Aqua dest.:Triäthanolamin im Verhältnis 80:10:10:2:0,1. Durch dieses Verfahren gewinnt man ca. 50 mg der halbreinen Fraktion A, die Kesarirhodin A enthält, und ca. 60 mg der halbreinen Fraktion B, die Kesarirhodin B enthält.

Die halbreinen Fraktionen werden zuerst mit Petroläther gewaschen und dann weiter getrennt gereinigt durch präparative Dünnschichtchromatographie auf Kieselgel mit Hilfe eines Lösungsmittelsystems, das aus Chloroform:Methanol:Essigsäure:Aqua dest.:Triäthanolamin im Verhältnis 80:10:10:2,0:0,1 besteht. Man erhält 27 mg der Substanz Kesarirhodin A und 32 mg der Substanz Kesarirhodin B.

Charakterisierung von Kesarirhodin A und B

Die reinen Substanzen Kesarirhodin A und B werden jeweils mittels chemischer Analyse und spektroskopischer Methoden analysiert.

Für UV-Absorptionsmaxima wird die Substanz in einer Konzentration von 10–30 mg/Liter verwendet. Das Absorptionsspektrum wurde für den Bereich von 200 bis 800 nm aufgezeichnet.

Die Protonenresonanzspektren ($^1H$-NMR-Spektren) wurden auf einem HX-270 BRUKER Fourier-Transform Magnetic Resonance Spektrometer bei 270 MHz aufgenommen. Die Konzentration der Probe betrug 2,6–2,7 mg/0,5 ml 99,8% $CDCl_3$, versetzt mit 0,1 ml 5% $Na_2CO_3$ in 99,5% $D_2O$.

Das Massenspektrum wurde in einem MS-902S, AEI, Massenspektrometer bestimmt (FAB (Fast-Atom-Bombardment)-Positivionen-Methode).

Physikalisch-chemische Eigenschaften von Kesarirhodin A
Aussehen: Orange-rotes Pulver
Chemische Formel: $C_{40}H_{53}O_{14}N$
Molekulargewicht: 771

UV-Spektrum:
λ max in:

(a) Methanol: 203, 234, 253, 292, 464, (sh), 478 (sh), 492, 512 (sh), 526, 575 nm.

(b) 0,1 N-HCl-Methanol: 203, 234, 253, 292, 464 (sh), 478 (sh), 492, 512 (sh), 526 (sh), 558 (sh) nm.

(c) 0,1 N-NaOH-Methanol: 203, 239, 298, 554, 586 nm.

$^1H$-NMR-Spektrum ($CDCl_3$ + 0,1 ml 5% $Na_2CO_3$ in $D_2O$): 7,88 (d, J=7 Hz, 1H), 7,69 (t, J=8 Hz, 1H), 7,29 (d, J=3 Hz, 1H), 5,49 (d, J=3 Hz, 1H) 5,21 (s, 1H), 5,03 (d, J=3 Hz, 1H), 4,85 (s, 1H), 4,51 (q, J=6 Hz, 1H) 4,21 (q, J=6 Hz, 1H), 4,0–4,1 (Komplex, 2H), 3,72 (s, 1H), 3,65 (s, 1H), 3,56 (s, 1H),3,25 (d, J=20 Hz, 1H), 2,56 (d, J=20 Hz, 1H), 2,34 (bd, J=14 Hz, 1H), 2,15 (s, 6H) 2,0–2,2 (Komplex, 4H), 1,61–1,9 (Komplex 8H), 1,17–1,32 (überlappende Dubletten, 6H), 1,14 (d, J=6 Hz, 3H), 1,07 (t, J=7,5 Hz, 3H).

Löslichkeit: Kesaririhodin A ist in Methanol, Aceton, Äthylacetat, Chloroform und schwachen Säuren löslich, in Hexan und Petroläther wenig löslich. Es bildet in Methanol eine orange-rote Lösung, nimmt aber im alkalischen Milieu eine purpurrote Farbe an.

Dünnschichtchromatgoraphie: Kesarirhodin A hat auf Kieselgelplatten mit verschiedenen Lösungsmittelsystemen die in Tabelle I dargestellten $R_f$-Werte.

Die vorgenannten Werte ergeben dass Kesarirhodin A die Strukturformel Ia besitzt.

Physikalisch-chemische Eigenschaften von Kesarirhodin B
Aussehen: Orange-rotes Pulver.
Chemische Formel: $C_{40}H_{53}O_{13}N$
Molekulargewicht: 755.

UV-Spektrum
λ max in:

(a) Methanol: 203, 234, 253, 292, 464, (sh), 478 (sh), 492, 510 (sh), 526, 582 nm.

(b) 0,1 N-HCl-Methanol: 203, 234, 253, 292, 464 (sh), 478 (sh), 492, 510 (sh), 526 (sh), 558 (sh) nm.

(c) 0,1 N-NaOH-Methanol: 203, 239, 298, 552, 584 nm.

$^1H$-NMR-Spektrum ($CDCl_3$+0,1 ml 5% $Na_2CO_3$ in $D_2O$): 7,88 (d, J=7 Hz, 1H), 7,7 (t, J=8 Hz, 1H) 7,31 (d, J=8 Hz, 1H), 5,52 (d, J=3 Hz, 1H), 5,21 (s, 1H), 4,95 (s, 1H), 4,83 (d,

J=3 Hz, 1H), 4,4 (q, J=6 Hz, 1H), 3,98–4,08 (Komplex, 2H) 3,78 (s, 1H), 3,56 (s, 1H), 3,46 (s, 1H), 3,26 (d, J=20 Hz, 1H), 2,58 (d, J=20 Hz, 1H), 2,25–2,38 (Komplex, 1H), 2,28 (s, 6H), 1,88–2,2 (Komplex, 4H), 1,5–1,85 (Komplex, 10H), 1,02–1,35 (überlappende Multipletts, 12H).

Löslichkeit: Kesarirhodin B ist in Methanol, Aceton, Äthylacetat, Chloroform und schwachen Säuren löslich, in n-Hexan und Petroleumäther wenig löslich. Es bildet in Methanol eine orangerote Lösung, nimmt im alkalischen Milieu hingegen eine purpurrote Farbe an.

Dünnschichtchromatographie: Kesarirhodin B hat auf Kieselgel-Platten bei Verwendung verschiedener Lösungsmittelsysteme die in Tabelle I angegebenen $R_f$-Werte.

Die vorgenannten Resultate ergeben, dass Kesarirhodin B die Strukturformel Ib besitzt.

Tabelle I: $R_f$-Werte Dünnschichtchromatographie (Verwendung von 0,2 mm Fertigplatten (Kieselgel auf Aluminiumfolie) (Kieselsäuregel 60 $F_{254}$ (Merck) )

| Lösungsmittelsystem | $R_f$-Werte Kesarirhodin A | Kesarirhodin B |
|---|---|---|
| Chloroform:Methanol (9:1) | 0,44 | 0,45 |
| Chloroform:Methanol:Essigsäure:Aqua dest.:Triäthanolamin (80:10:10:2,0:0,1) | 0,51 | 0,75 |
| Äthylacetat:Methanol (9:1) | 0,21 | 0,21 |

Antibakterielle Wirkung von Kesarirhodin

Bestimmt wurde die bakteriostatische Wirkung von Kesarirhodin. Die Resultate, die als Durchmesser (in Millimetern) der Hemmhöfe, erzeugt durch die Substanz in Form einer 1 mg/ml-Lösung in einem Loch von 6 mm Durchmesser in Agar, das den Testorganismus enthält, angegeben sind, werden in der nachfolgenden Tabelle, Tabelle II, dargestellt. Kesarirhodine sind gegen gram-positive Bakterien und Pilze wirksam und können deshalb als Antibiotika zur Behandlung von Pilz- und Staphylokokken-Infektionen, Diphterie, Pneumonie usw. eingesetzt werden.

Tabelle II: Bakteriostatische Wirkung

| Mikroorganismus | Hofdurchmesser (mm) Kesarirhodin (1 mg/ml) A | B |
|---|---|---|
| Staphylococcus aureus 209P | 15 | 17 |
| Bacillus subtilis | 17 | 17 |
| Escherichia coli 9632 | – | – |
| Pseudomonas aeruginosa | – | – |
| Proteus vulgaris | – | – |
| Candida albicans | 20 | 20 |

Antitumor-Wirkung von Kesarirhodin

Die Ermittlung der zytostatischen Aktivität der Kesarirhodine erfolgte in der nachfolgend beschriebenen Weise an Mäuse-L 1210-Leukämiezellen.

(a) Proliferationstest

Bei dieser in vitro-Methode ist es nach der Inkubation der Zellen mit verschiedenen Konzentrationen der Prüfsubstanz möglich, die eingebaute Menge eines radioaktiv markierten DNS-Vorläufers (z.B. 14C-Thymidin) in einer wachsenden Zelle zu bestimmen. Als Kontrolle dienen dem gleichen Verfahren unterzogenen unbehandelte L 1210-Zellen. Die Methode wird nachstehend kurz zusammengefasst:

L 1210-Zellen in exponentieller Wachstumsphase ($5\times10^3$/ml in RPMI 1640) werden 72 Stunden lang (37 °C, 5% $CO_2$, 95% relative Feuchtigkeit) in einer 96-Loch-Mikrotiterplatte mit unterschiedlichen Konzentrationen der Prüfsubstanz inkubiert. Als Kontrollen werden Zellen verwendet, die lediglich mit dem frischen Medium in Berührung gebracht werden. Es werden vier Löcher für jede Medikamentenkonzentration und Kontrolle angelegt. Nach 65 Stunden werden 50 µl 14C Thymidin (1,5 µCi/ml) zugefügt, um die Zell-DNS zu markieren. Nach 7 Stunden Inkubation werden die Zellen geerntet, die DNS mit 5% Trichloressigsäure abgetrennt und mit Wasser und Methanol gewaschen. Nach dem Trocknen bei 50 °C wird die Szintillationszahl mit 5 ml Szintillationsflüssigkeit bestimmt.

Die Ergebnisse werden als Quotient des Szintillationsindex nach der Inkubation mit den Testsubstanzen mit dem der Kontrollsubstanz ausgedrückt. Mit den ermittelten Werten wird eine Dosiswirkungskurve gezeichnet und der Wert $IC_{50}$ d.h. die Konzentration der Prüfsubstanz, die den Einbau von markiertem Thymidin im Vergleich zur Kontrolle um 50% hemmt, graphisch ermittelt. Die $IC_{50}$-Werte sind denen von Adriamycin in der folgenden Tabelle III gegenübergestellt.

(b) Stammzellentest (Kontinuierliches Experiment in Weichagar)

Diese Methode dient zum Nachweis der Wirkung der Prüfsubstanz auf das Wachstum von Leukämiezellen über mehrere Generationen (da die Dauer eines Zellzyklus 10–12 Stunden beträgt, werden innerhalb des Untersuchungszeitraumes von 7 Tagen rund 14 aufeinanderfolgende Generationen erfasst).

In diesem Test beruht die Wirkung von zytotoxischen Substanzen auf der Verminderung der Zahl beobachtbarer Kolonien im Vergleich zur unbehandelten Kontrolle. Es wird folgende Methodik verwendet:

Pro Platte werden 500 L 1210-Leukämiezellen verwendet. Die Experimente werden bei kontinuierlicher Inkubation mit verschiedenen Konzentrationen der Prüfsubstanz durchgeführt, wobei die Substanz vor dem Aufbringen der Zellen auf die Platte der oberen Agarschicht beigemischt wird. Die Platten werden 7 Tage lang in einem Brutschrank gelagert (37 °C, 5% $CO_2$, 95% relative Luftfeuchtigkeit). Anschliessend wird die Anzahl der entstandenen Kolonien mit einem Durchmesser von mehr als 60 µm mit einem umgekehrten Mikroskop gezählt. Die Resultate werden als Prozentsatz der auf den behandelten Platten gebildeten Kolonien im Vergleich zu denen der unbehandelten Kontrolle ausgedrückt. Mit den ermittelten Werten wird eine Dosiswirkungskurve gezeichnet, aus welcher der $IC_{50}$-Wert graphisch ermittelt wird. Die $IC_{50}$-Werte der Kesarirhodine werden in Tabelle III mit denen von Adriamycin verglichen.

Tabelle III: Antitumor-Aktivität

| Substanz | $IC_{50}$ (µg/ml) Proliferationstest | Stammzellentest 7 Tage Inkubation |
|---|---|---|
| Adriamycin | $6{,}0\times10^{-3}$ | $2{,}2\times10^{-2}$ |
| Kesarirhodin A | $3{,}1\times10^{-2}$ | $3{,}1\times10^{-2}$ |
| Kesarirhodin B | 0,1 | $2{,}8\times10^{-2}$ |

Wie vorstehend ausgeführt, besitzen die erfindungsgemässen Verbindungen antibakterielle, fungizide und zytostatische Wirksamkeit, d.h. therapeutische Wirkung, insbesondere gegen maligne Tumoren bei Tieren und Menschen.

Die Verbindungen und die Säureadditionssalze können daher als Medikamente zur Behandlung von Infektionskrankheiten, Pilzerkrankungen und von Tumoren verwendet werden. Die Verbindungen können auf verschiedene Weise in Abhängigkeit von der Dosierungsform verabreicht werden. Normalerweise werden die Verbindungen mit pharmazeutisch üblichen Trägerstoffen oder Verdünnungsmitteln vermischt verabreicht. So können sie z.B. einzeln oder in Mischung zusammen mit Trägerstoffen wie Maltose oder Lactose oder als nichttoxische Komplexe, z.B. als Desoxyribonukleinsäure-Komplex verabreicht werden.

Eine typische Verabreichungsart ist die Injektion einer Lösung der erfindungsgemässen Verbindungen in destilliertem Wasser oder in physiologischer Kochsalzlösung. Die Lösungen können intraperitoneal, intravenös oder intraarteriell injiziert werden.

Tagesdosis und Einheitsdosis können aus Tierversuchen und auch aus in vitro-Tests in der Weise festgesetzt werden, dass die Gesamtdosis, die kontinuierlich oder in Abständen verabreicht wird, einen vorher festgelegten Bereich nicht überschreitet. So beträgt die Gesamtdosis für einen Behandlungszyklus bei Krebserkrankungen etwa 0,1–20 mg, bevorzugt 0,5–10 mg/kg Körpergewicht. Diese Dosis kann in entsprechenden Bruchteilen über einen Zeitraum von 7 Tagen verabreicht werden. Für die Behandlung von Infektions- und Pilzerkrankungen kommt als Dosiseinheit 0,1–10 mg, bevorzugt 0,5–5 mg/kg Körpergewicht in Betracht. Als Tagesdosis kann die ein- bis dreifache Dosiseinheit verabreicht werden.

**Patentansprüche für die Vertragsstaaten BE, CH, DE, FR, GB, IT, LI, LU, NL, SE**

1. Verbindungen der Formel I

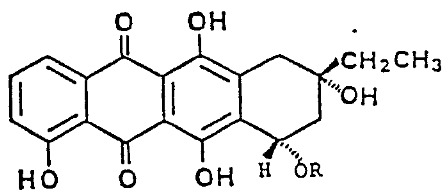

I

worin R eine Zucker-Kombination der Zusammensetzung Roa-dF-Rod oder Roa-Rod-Rod bedeutet, wobei Roa Rhodosamin, dF Desoxyfucose und Rod Rhodinose darstellt, sowie deren physiologisch unbedenklichen Säureadditionssalze.

2. Verfahren zur Herstellung von Verbindungen der Formel I

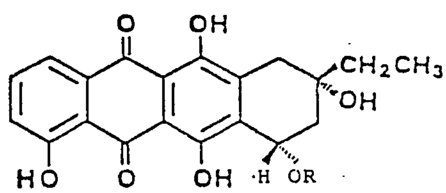

worin R eine Zucker-Kombination der Zusammensetzung Roa-dF-Rod oder Roa-Rod-Rod bedeutet, wobei Roa Rhodosamin, dF Desoxyfucose und Rod Rhodinose darstellt, sowie deren physiologisch unbedenklichen Säureadditionssalze, dadurch gekennzeichnet, dass man den Mikroorganismus Streptomyces purpurascens DSM 2658 auf übliche Weise unter aeroben Bedingungen in Gegenwart eines Nährmediums fermentiert, nach einer bestimmten Fermentationsdauer einen chemischen Inhibitor zusetzt und die gebildeten Verbindungen aus der Kulturflüssigkeit und dem Mycel mit organischen Lösungsmitteln und anschliessender Chromatographie in üblicher Weise isoliert.

3. Verfahren nach Anspruch 2, dadurch gekennzeichnet, dass man als chemischen Inhibitor Kaliumcyanid, Kaliumhexacyanoferrat (III), Natriumazid, Eisen(III)chlorid, Methylenblau, Triphenyltetrazoliumchlorid, Sulfanilsäure, Sulfanilamid, Ascorbinsäure und deren Salze, Äthylendiamintetraessigsäure, Nitrofurazon, Salinomycin, Dithionit, Rotenon, 2,4-Dinitrophenol, Erythromycin oder Dimethylsulfoxid verwendet.

4. Verfahren nach Anspruch 2 oder 3, dadurch gekennzeichnet, dass man als chemischen Inhibitor Eisen(III)chlorid, Natriumazid oder Ascorbinsäure verwendet, und diesen nach einer Fermentationsdauer von 20–36 Stunden zusetzt.

5. Pharmazeutische Zubereitung dadurch gekennzeichnet, dass sie eine Verbindung gemäss

Anspruch 1 neben einem festen, flüssigen oder halbflüssigen Träger- oder Hilfsstoff enthält.

6. Verwendung einer Verbindung gemäss Anspruch 1 zur Herstellung von Medikamenten, die zur Behandlung von bakteriellen Infektionskrankheiten, Pilz- und Krebserkrankungen geeignet sind.

**Patentansprüche für den Vertragsstaat AT**

1. Verfahren zur Herstellung von Verbindungen der Formel I

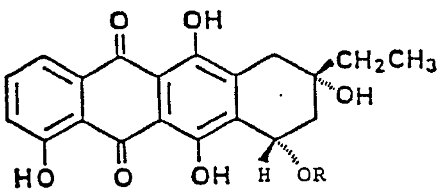

worin R eine Zucker-Kombination der Zusammensetzung Roa-dF-Rod oder Roa-Rod-Rod bedeutet, wobei Roa Rhodosamin, dF Desoxyfucose und Rod Rhodinose darstellt, sowie deren physiologisch unbedenklichen Säureadditionssalze, dadurch gekennzeichnet, dass man den Mikroorganismus Streptomyces pupurascens DSM 2658 auf übliche Weise unter aeroben Bedingungen in Gegenwart eines Nährmediums fermentiert, nach einer bestimmten Fermentationsdauer einen chemischen Inhibitor zusetzt und die gebildeten Verbindungen aus der Kulturflüssigkeit und dem Mycel mit organischen Lösungsmitteln und anschliessender Chromatographie in üblicher Weise isoliert.

2. Verfahren nach Anspruch 1, dadurch gekennzeichnet, dass man als chemischen Inhibitor Kaliumcyanid, Kaliumhexycyanoferrat (III), Natriumazid, Eisen(III)chlorid, Methylenblau, Triphenyltetrazoliumchlorid, Sulfanilsäure, Sulfanilamid, Ascorbinsäure und deren Salze, Äthylendiamintetraessigsäure, Nitrofurazon, Salinomycin, Dithionit, Rotenon, 2,4-Dinitrophenol, Erythromycin oder Dimethylsulfoxid verwendet.

3. Verfahren nach Anspruch 1 oder 2, dadurch gekennzeichnet, dass man als chemischen Inhibitor Eisen(III)chlorid, Natriumazid oder Ascorbinsäure verwendet, und diesen nach einer Fermentationsdauer von 20–36 Stunden zusetzt.

**Revendications pour les Etats Contractants BE, CH, DE, FR, GB, IT, LI, LU, NL, SE**

1. Composés de formule I

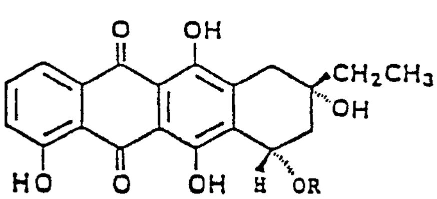 I

dans laquelle R représente une combinaison de sucres de composition Roa-dF-Rod ou Roa-Rod-Rod, Roa représentant la rhodosamine, dF le désoxyfucose et Rod le rhodinose, ainsi que leurs sels d'addition avec des acides physiologiquement acceptables.

2. Procédé pour la préparation de composés de formule I

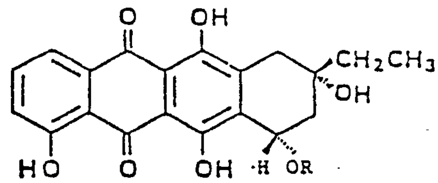

dans laquelle R représente une combinaison de sucres de composition Roa-df-Rod ou Roa-Rod-Rod, Roa représentant la rhodosamine, dF le désoxyfucose et Rod le rhodinose, ainsi que des sels d'addition avec des acides physiologiquement acceptables de ceux-ci, caractérisé en ce que l'on effectue une fermentation de façon usuelle à partir du micro-organisme Streptomyces purpurascens DSM 2658, dans les conditions aérobies, en présence d'un milieu nutritif, on ajoute un inhibiteur chimique après une durée déterminée de fermentation, et à partir du liquide de culture et du mycélium, on isole de façon usuelle, à l'aide de solvants organiques et de chromatographie subséquente, les composés formés.

3. Procédé selon la revendication 2, caractérisé en ce que, en tant qu'inhibiteur chimique, on utilise le cyanure de potassium, l'hexacyanoferrate (III) de potassium, l'azoture de sodium, le chlorure ferrique, le bleu de méthylène, le chlorure de triphényltétrazolium, l'acide sulfanilique, le sulfanilamide, l'acide ascorbique et ses sels, l'acide éthylènediaminetétraacétique, la nitrofurazone, la salinomycine, le dithionite, la roténone, le 2,4-dinitrophénol, l'érythromycine ou le diméthylsulfoxyde.

4. Procédé selon la revendication 2 ou 3, caractérisé en ce que, en tant qu'inhibiteur chimique, on utilise le chlorure ferrique, l'azoture de sodium ou l'acide ascorbique, et on l'ajoute après une durée de fermentation de 20 à 36 heures.

5. Composition pharmaceutique, caractérisée en ce qu'elle contient un composé selon la revendication 1, en plus d'un véhicule ou excipient solide, liquide ou semi-liquide.

6. Utilisation d'un composé selon la revendication 1, pour la fabrication de médicaments qui sont appropriés au traitement de maladies infectieuses bactériennes, de maladies fongiques et de maladies cancéreuses.

**Revendications pour l'Etat Contractant AT**

1. Procédé pour la préparation de composés de formule I

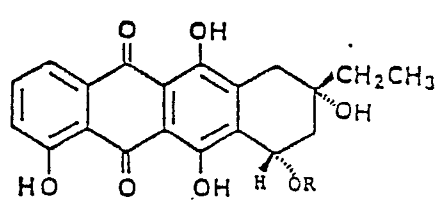

dans laquelle R représente une combinaison de sucres de composition Roa-df-Rod ou Roa-Rod-Rod, Roa représentant la rhodosamine, dF le désoxyfucose et Rod le rhodinose, ainsi que des sels d'addition avec des acides physiologiquement acceptables de ceux-ci, caractérisé en ce que l'on effectue une fermentation de façon usuelle à partir du micro-organisme Streptomyces purpurascens DSM 2658, dans les conditions aérobies, en présence d'un milieu nutritif, on ajoute un inhibiteur chimique après une durée déterminée de fermentation, et à partir du liquide de culture et du mycélium, on isole de façon usuelle, à l'aide de solvants organiques et de chromatographie subséquente, les composés formés.

2. Procédé selon la revendication 1, caractérisé en ce que, en tant qu'inhibiteur chimique, on utilise le cyanure de potassium, l'hexacyanoferrate (III) de potassium, l'azoture de sodium, le chlorure ferrique, le bleu de méthylène, le chlorure de triphényltétrazolium, l'acide sulfanilique, le sulfanilamide, l'acide ascorbique et ses sels, l'acide éthylènediaminetétraacétique, la nitrofurazone, la salinomycine, le dithionite, la roténone, le 2,4-dinitrophénol, l'érythromycine ou le diméthylsulfoxyde.

3. Procédé selon la revendication 1 ou 2, caractérisé en ce que, en tant qu'inhibiteur chimique, on utilise le chlorure ferrique, l'azoture de sodium ou l'acide ascorbique, et on l'ajoute après une durée de fermentation de 20 à 36 heures.

**Claims for the Contracting States BE, CH, DE, FR, GB, IT, LI, LU, NL, SE**

1. A Compound of the formula I

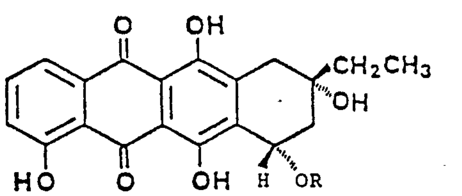

I

in which R represents a sugar combination of the composition Roa-dF-Rod or Roa-Rod-Roo, where Roa represents rhodosamine, dF represents deoxyfucose and Rod represents rhodinose, and the physiologically acceptable acid addition salts thereof.

2. A process for the preparation of compounds of the Formula I

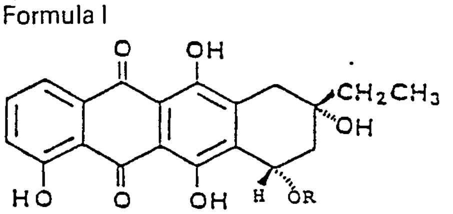

in which R represents a sugar combination of the composition Roa-dF-Rod or Roa-Rod-Rod, where Roa represents rhodosamine, dF represents deoxyfucose and Rod represents rhodinose, and the physiologically acceptable acid addition salts thereof, which comprises fermentation of the microorganism Streptomyces purpurascens DSM 2658 in a customary manner under aerobic conditions, in the presence of a nutrient medium, addition of a chemical inhibitor after a particular fermentation time, and isolation, from the culture liquid and the mycelium, of the compounds which have been formed, using organic solvents followed by chromatography in a customary manner.

3. The process as claimed in claim 2, wherein the chemical inhibitor used is potassium cyanide, potassium hexacyanoferrate(III), sodium azide, iron(III) chloride, methylene blue, triphenyltetrazolium chloride, sulfanilic acid, sulfanilamide, ascorbic acid and its salts, ethylenediaminetetraacetic acid, nitrofurazone, salinomycin, dithionite, rotenone, 2,4-dinitrophenol, erythromycin or dimethyl sulfoxide.

4. The process as claimed in claim 2 or 3, wherein the chemical inhibitor used is iron(III) chloride, sodium azide or ascorbic acid, and this is added after a fermentation time of 20–36 hours.

5. A pharmaceutical formulation which contains a compound as claimed in claim 1, in addition to a solid, liquid or semiliquid vehicle or auxiliary.

6. The use of a compound as claimed in claim 1 for the preparation of medicaments which are suitable for the treatment of bacterial infectious diseases, and fungal and cancerous diseases.

**Claims for the contracting State AT**

1. A process for the preparation of compounds of the Formula I

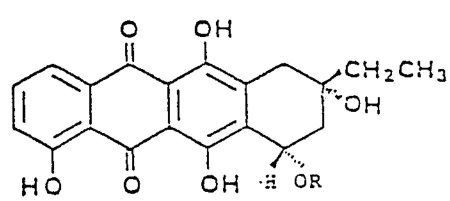

in which R represents a sugar combination of the composition Roa-dF-Rod or Roa-Rod-Rod, where Roa represents rhodosamine, dF represents deoxyfucose and Rod represents rhodinose, and the physiologically acceptable acid addition salts thereof, which comprises fermentation of the microorganism Streptomyces purpurascens DSM 2658 in a customary manner under aerobic conditions, in the presence of a nutrient medium, addition of a chemical inhibitor after a particular fermentation time, and isolation, from the culture liquid and the mycelium, of the compounds which have been formed, using organic solvents followed by chromatography in a customary manner.

2. The process as claimed in claim 1, wherein the chemical inhibitor used is potassium cynide, potassium hexacyanoferrate(III), sodium azide, iron(III) chloride, methylene blue, triphenyltetrazolium chloride, sulfanilic acid, sulfanilamide, ascorbic acid and its salts, ethylenediaminetetraacetic acid, nitrofurazone, salinomycin, dithionite, rotenone, 2,4-dinitrophenol, erythromycin or dimethyl sulfoxide.

3. The process as claimed in claim 1 or 2, wherein the chemical inhibitor used is iron(III) chloride, sodium azide or ascorbic acid, and this is added after a fermentation time of 20–36 hours.